(19) Europäisches Patentamt
European Patent Office
Office européen des brevets

(11) **EP 4 576 102 A1**

(12) **EUROPEAN PATENT APPLICATION**

(43) Date of publication:
**25.06.2025 Bulletin 2025/26**

(21) Application number: **24222357.6**

(22) Date of filing: **20.12.2024**

(51) International Patent Classification (IPC):
**G16B 40/20** $^{(2019.01)}$       **G16B 30/00** $^{(2019.01)}$

(52) Cooperative Patent Classification (CPC):
**G16B 40/20; G16B 30/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.12.2023 CN 202311787490**

(71) Applicant: **GeneMind Biosciences Company
Limited
Shenzhen City, 518023 Guangdong (CN)**

(72) Inventors:
• **LIU, Yurun
Shenzhen City, 518023 (CN)**
• **CHEN, Weiyue
Shenzhen City, 518023 (CN)**

(74) Representative: **Schiweck Weinzierl Koch
Patentanwälte Partnerschaft mbB
Ganghoferstraße 68 B
80339 München (DE)**

(54) **METHOD AND APPARATUS FOR DETERMINING QUALITY PARAMETER IN BASECALL**

(57)   The present application discloses a method and an apparatus for determining a quality parameter in basecall. The method comprises: acquiring calling result data of a basecall model for a nucleic acid sample of interest, the calling result data comprising base probability distribution for a current base extension reaction in the nucleic acid sample of interest; calculating a first parameter on the basis of the base probability distribution; and filtering the first parameters on the basis of a preset $Q_0$ value to give a target quality parameter. In the present application, the target quality parameter is determined using the information output by the basecall model, such that the target quality parameter can more accurately determine the basecall results learned by a machine.

FIG. 1

## Description

## TECHNICAL FIELD

The present application relates to the technical field of data processing, in particular to a method and an apparatus for determining a quality parameter in basecall.

## BACKGROUND

A variety of data are present in the gene sequencing result file, including at least two important data: the basecall result data and the quality score data. The quality score is typically expressed by a Q value, which exists in the sense that each identified or output base is scored to determine the reliability of that base. It can be seen that the application accuracy of the quality score Q value can influence the final basecall result.

## SUMMARY

In view of this, the present application provides the following technical solutions:
A method for determining a quality parameter in basecall is provided, comprising:

> acquiring calling result data of a basecall model for a nucleic acid sample of interest, the calling result data comprising base probability distribution for a current base extension reaction in the nucleic acid sample of interest;
> calculating a first parameter on the basis of the base probability distribution; and filtering the first parameters on the basis of a preset Qo value to give a target quality parameter, wherein $Qo = -10 \times \log_{10}e$, and e is a basecall error rate.

An apparatus for determining a quality parameter in basecall is further provided, comprising:

> an acquisition unit, configured for acquiring calling result data of a basecall model for a nucleic acid sample of interest, the calling result data comprising base probability distribution for a current base extension reaction in the nucleic acid sample of interest;
> a calculation unit, configured for calculating a first parameter on the basis of the base probability distribution; and
> a processing unit, configured for filtering the first parameters on the basis of a preset Qo value to give a target quality parameter, wherein $Qo = -10 \times \log_{10}e$, and e is a basecall error rate.

It can be seen from the technical solutions that the present application discloses a method and an apparatus for determining a quality parameter in basecall.

The method comprises: acquiring calling result data of a basecall model for a nucleic acid sample of interest, the calling result data comprising base probability distribution for a current base extension reaction in the nucleic acid sample of interest; calculating a first parameter on the basis of the base probability distribution; and filtering the first parameters on the basis of a preset $Q_0$ value to give a target quality parameter. In the present application, the target quality parameter is determined using the information output by the basecall model, such that the target quality parameter can more accurately determine the basecall results learned by a machine.

## BRIEF DESCRIPTION OF THE DRAWINGS

In order to more clearly illustrate the technical solutions in the examples of the present application, the drawings required for use in the description of the examples will be briefly described below. It is obvious that the drawings in the description below are only examples of the present application, and other drawings can be derived from the provided drawings by those of ordinary skill in the art without making inventive efforts.

FIG. 1 is a schematic flowchart of a method for determining a quality parameter in basecall according to the examples of the present application;
FIG. 2 is a schematic of the correspondence curve of one error rate and $Q_1$ according to the examples of the present application;
FIG. 3 is a schematic of the correspondence curve of another error rate and target $Q_1$ according to the examples of the present application;
FIG. 4 is a schematic of the correspondence curve of yet another error rate and target $Q_1$ according to the examples of the present application;
FIG. 5 is a schematic of the error rate curve comparison of aligned sequences in sequences filtered by Qo and $Q_T$ according to the examples of the present application;
FIG. 6 is a schematic of the loss curve comparison in sequences filtered by $Q_0$ and $Q_T$ according to the examples of the present application;
FIG. 7 is a schematic of the overall sequence loss curve comparison in sequences separately filtered by Qo and $Q_T$ according to the examples of the present application;
FIG. 8 is a schematic of curves illustrating the related information of one target quality parameter $Q_T$ and the error rate according to the examples of the present application;
FIG. 9 is a schematic of one error rate decreasing curve according to the examples of the present application;
FIG. 10 is a schematic of another error rate decreasing curve according to the examples of the present application;
FIG. 11 is a schematic of the retained data error rate curves after one filtering according to the examples of the present application;

FIG. 12 is a schematic of the retained error rate curves after another filtering according to the examples of the present application;

FIG. 13 is a structural schematic of an apparatus for determining a quality parameter in basecall according to the examples of the present application.

DETAILED DESCRIPTION

The technical solutions in the examples of the present application will be described below clearly and comprehensively in conjunction with the drawings in the examples of the present application. It is obvious that the described examples are part of the examples of the present application, but not all of them. On the basis of the examples of the present application, all other examples obtained by those of ordinary skill in the art without inventive efforts shall fall within the protection scope of the present application.

The terms "first", "second", and the like in the specification and claims of the present application and in the aforementioned drawings, are used for distinguishing between different objects but not for describing a particular order. Furthermore, the terms "comprise" and "have" as well as any variations thereof, are intended to encompass a non-exclusive inclusion. For example, a process, method, system, product, or device that comprises a list of procedures or units is not limited to the listed procedures or units but may include other procedures or units not listed.

In the embodiments of the present application, the term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a template and acquiring the corresponding signals emitted by the nucleotides (including analogs). The so-called sequencing includes sequencing by synthesis (SBS) and/or sequencing by ligation (SBL), DNA sequencing and/or RNA sequencing, and long fragment sequencing and/or short fragment sequencing (the long fragment and short fragment are defined relatively; e.g., nucleic acid molecules longer than 1 Kb, 2 Kb, 5 Kb, or 10 Kb may be referred to as long fragments, and nucleic acid molecules shorter than 1 Kb or 800 bp may be referred to as short fragments). The sequencing generally involves cycles of process to achieve the determination of the type and order of multiple bases or nucleotides on a nucleic acid template. The examples of the present application refer to each cycle of the "process to achieve the determination of the type and order of multiple bases or nucleotides on a nucleic acid template" as "one cycle of sequencing". The "cycle of sequencing", also referred to as "sequencing cycle", may be defined as the completion of one base extension of four types of nucleotides/bases; in other words, the "cycle of sequencing" may be defined as the determination of the base or nucleotide type at any given position on the template. For sequencing platforms that achieve sequencing on the basis of polymerization or ligation reactions, the cycle of sequencing includes the process of binding four types of nucleotides (including nucleotide analogs) to the nucleic acid template at a time through base complementation and acquiring the corresponding signals emitted. For platforms that achieve sequencing on the basis of polymerization reaction, a reaction system includes reaction substrate nucleotide, polymerase, and a nucleic acid template. A sequence fragment (a sequencing primer) binds to the nucleic acid template, and on the basis of the base pairing rules and the principle of polymerization reaction, the added reaction substrate nucleotides are connected to the sequencing primer under the catalysis of the polymerase to realize the binding of a nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension.

In the examples of the present application, the term "channel" refers to four channels formed in different ways during the sequencing process for screening and distinguishing four bases derived from A, C, G, and T or U. For example, the channel may refer to four fluorescent signal optical channels formed by using different excitation lights, different fluorescence filters, and the like in the photographing process of sequencing for screening and distinguishing the four fluorescent bases derived from A, C, G, and T or U. In the practice of sequencing, fluorescent images are obtained by taking pictures of the four different fluorescence channels. Ideally, each fluorescence channel only contains the signal of the fluorescent base type corresponding to the channel, but in practical cases, due to the influence of fluorescence crosstalk, the fluorescent signals of other bases may also be present in each channel besides the fluorescent signal of the corresponding fluorescent base.

A variety of data are present in the gene sequencing result file, including at least two important data: the basecall result data and the quality score data. The quality score is typically expressed by a Q value. The Q value exists in the sense that each identified or output base is scored to determine the reliability of that base. Currently, when a basecall is performed on the basis of fluorescent signals, the parameter Q value is characterized by a parameter for evaluating the base reliability obtained by summarizing optical data corresponding to the four bases in a current base extension reaction.

For example, the second-generation sequencing technology utilizes the characteristic that different fluorescent molecules have different fluorescence emission wavelengths and adopts different fluorescent molecules to label the substrates for base extension reaction. After the base extension reaction, laser irradiates the fluorescent molecules to excite the fluorescent molecules to generate fluorescent signals. The fluorescent signals with specific wavelengths are acquired through an optical sensor, and the types of the bases in the bound fluorescent molecules are identified on the basis of the fluorescent signals. For example, in two-color sequencing, four different fluorescent labels are adopted to respectively label four different bases (A, T or U, G, and C). The four bases are added to complete one base extension reaction. The fluorescent molecules are excited by laser irradiation to generate fluorescent signals, and images of the fluorescent signals generated by excitation with different excitation wavelengths are acquired by an optical imaging system. In one mode, the fluorescent images are subjected to image processing and fluorescence point positioning to detect the base cluster. According to the base cluster detection results of a plurality of fluorescent images corresponding to sequencing signal responses of different base types, templates are constructed, and the positions of all base cluster template points (clusters) are established. According to the template, the filtered images are subjected to optical data extraction (typically, fluorescence intensity extraction), and the fluorescence intensity is corrected. Finally, bases are identified according to the maximum intensity of the position of each cluster, and the score is calculated, so as to give a base sequence file.

In each cycle of sequencing, every identified base has a quality score for evaluating the accuracy of the identification result, known as the quality score (generally expressed by a Q value or Qphred). The quality score indicates the reliability and error rate (e) of basecall in the sequencing process. The Q value is calculated as follows: $Qphred = -10 \times \log_{10}e$, and as can be seen from this equation, a greater Q value indicates a lower possibility of calling errors and a higher reliability. Commonly used Q values in statistics correspond to different error rates. In high-throughput sequencing, Q20 can be used as a threshold for base filtering, and Q30 is also frequently used to assess the quality of sequencing. Q20 indicates that in the basecall result output by sequencing, the probability of a correctly recognized base is 99% and the probability of an incorrectly recognized base is 1%; Q30 corresponds to that in the basecall result output by sequencing, the probability of a correctly recognized base is 99.9% and the probability of an incorrectly recognized base is 0.1%.

In a conventional sequencing method via fluorescent signal-based basecalls, the algorithm of quality score (for distinguishing from the quality parameters in the examples of the present application, the quality score is hereinafter referred to as a Qo value) is to perform error rate summarization on the basis of the numerical intensities of the fluorescent lights of the four bases in fluorescent images, thereby giving a Qo value. This algorithm has been verified with strict error rate distribution statistics and is therefore a relatively standard Q value compared with the bases of the conventional basecalls. At present, with the development of base call technology, more and more algorithms for basecalls based on machine learning are available. The error rates of bases output by basecall algorithms based on machine learning are lower than those of conventional basecalls, leading to effectively reduced error rates and improved base quality. However, the way of outputting bases by algorithms does not completely depend on the fluorescence intensity information of the four bases in the fluorescent images. As such, the use of the original quality parameter (Qo value) cannot well reflect the error rate information of bases output by basecalls based on machine learning.

In view of this, the examples of the present application provide a method for determining a quality parameter in a basecall algorithm applicable to machine learning, so as to better evaluate the accuracy of basecalls based on machine learning.

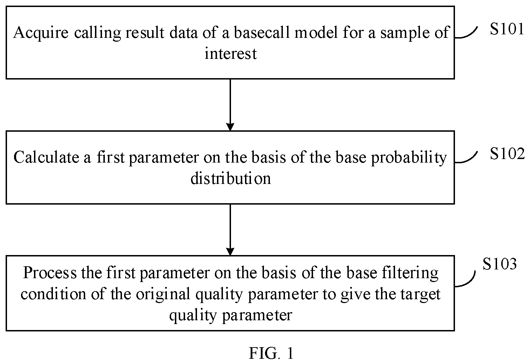

FIG. 1 is a schematic flowchart of a method for determining a quality parameter in basecall according to the examples of the present application. The method comprises:

S 101, acquiring calling result data of a basecall model for a nucleic acid sample of interest.

In the examples of the present application, the output of the basecall model is image data formed by one or more fluorescent images generated on the basis of the sequencing signals of the bases participating in the base extension reaction. The fluorescent image may be an original fluorescent image captured on the surface of the sequencing chip where the nucleic acid sample of interest is positioned in each cycle of sequencing, or a corrected fluorescent image acquired by correcting the original fluorescent image captured on the surface of the sequencing chip where the nucleic acid sample of interest is positioned in each cycle of sequencing. The examples of the present application extract the features of multi-channel input images through the network result of the basecall model, and determine the basecall result corresponding to the input image data of each channel on the basis of the extracted features, thereby giving the calling result data. The basecall result may have different presentations. For example, the base type corresponding to the current base extension reaction position or site is directly output, or the basecall result may be characterized in the form of probability data of each recognized base. In one embodiment, the extracted features may include at least one of the following: the combination of the basecall results of the previous cycle of sequencing, the current cycle of sequencing, and the next cycle of sequencing, the four fluorescence brightness values of the fluorescent image of the current cycle of sequencing, the ratio of the maximum value to the second maximum value among the four fluorescence brightness values of the fluorescent image of the current cycle of sequencing, and the standard deviation of the four fluorescence brightness values of the fluorescent image of the current cycle of sequencing. In one embodiment, the basecall result may comprise base types output by signals based on positions of the base extension reaction or probability data of each identified base in the fluorescent image. Specifically, the probability data comprises base probability distribution for a current base extension reaction in the nucleic acid sample of interest. In the present example, the base probability distribution refers to the probability that the base participating in the base extension reaction is identified as A, T (or U), G, or C for a given cycle of base extension reaction. It will be appreciated that, for each current base extension reaction of the nucleic acid sample of interest, the sum of the probabilities of A, T (or U), G, and C is 1. For example, for the probability data at the positions of the acquisition centers of base signals indicating whether the base extension reaction position or site is a certain base type, the probability values at the positions of the acquisition centers of base signals denote the probability that the base signal is base type A, C, G, or T or U, and the sum of the probabilities of the four base types is 1.

S102, calculating a first parameter on the basis of the base probability distribution.

In the examples of the present application, the target quality parameter is determined by using the base probability distribution data. However, the base probability distribution is generally a percentage parameter, and in order to generate the target quality parameter for base evaluation more accurately, the acquired base probability distribution data needs to be converted to give the first parameter. That is, the first parameter is given by the calculation of the base probability distribution data in the corresponding data format. The first parameter is then processed on the basis of the preset Qo value to give the target quality parameter, such that the target quality parameter also possesses base filtering functionality similar to that of the Qo value. Specifically, when acquired by calculation on the basis of the base probability distribution, the first parameter may be acquired by processing the maximum probability parameter on the basis of the current base extension reaction, or processing a selected probability parameter meeting a specific condition.

In one embodiment, calculating the first parameter on the basis of the base probability distribution, comprises: acquiring a maximum probability parameter in the current base extension reaction on the basis of the base probability distribution; and calculating the first parameter on the basis of the maximum probability parameter. Furthermore, calculating the first parameter on the basis of the maximum probability parameter, comprises: determining a second parameter $Q_1$ on the basis of formula $Q_1 = -10 \times \log_{10}(1-P_{max})$, wherein $P_{max}$ denotes the maximum probability parameter; comparing the second parameter with a preset value, and if the second parameter is greater than the preset value, rounding the second parameter to the nearest integer toward zero to give the first parameter; and if the second parameter is not greater than the preset value, rounding the second parameter to the nearest integer to give the first parameter, wherein the range of the first parameter is positive integers from 0 to the preset value.

The method first separately summarizes the maximum value of the prediction probabilities corresponding to the bases of the prediction probabilities in the output result of the basecall model and finds the corresponding bases. As such, a value representing the base is acquired, and meanwhile, the value represents the corresponding maximum prediction probability value, with a higher probability indicating a higher reliability. Since the probability is usually expressed as a percentage, such as 75%, 60%, etc., in order to generate the target quality parameter for base evaluation more accurately, the probability value needs to be converted into a value within a preset range. That is, the probability value is converted into a value less than or equal to a preset value. In some examples, when the preset value is 100, the probability is converted to a value of 0-100 to give the target quality parameter. Specifically, the maximum probability parameter is first converted into a value for calculation in the determination of the target quality parameter, i.e., the second parameter $Q_1$, wherein $Q_1 = -10 \times \log_{10}(1-P_{max})$. The preset value is set as 100 since the final target quality parameter is an integer of 0-100. If the calculated second parameter is greater than 100, the second parameter is rounded to the nearest integer toward zero to give the first parameter; if the second parameter is not greater than 100, it is rounded to the nearest integer to give the first parameter.

S103, filtering the first parameters on the basis of a preset Qo value to give a target quality parameter.

After acquiring the first parameter, the first parameter may be a value of 0-100. By comparing with the preset Qo value, the error rate of the value of 0 to 100 may be summarized according to the definition of the $Q_0$ value and the segmentation criteria to assign a new target quality parameter, so as to give the target quality parameter applicable to basecall result evaluation in the basecall model according to the examples of the present application. As described above, in the examples of the present application, $Q_0 = -10 \times \log_{10}e$, and e is a basecall error rate.

In one embodiment, filtering the first parameters on the basis of the preset Qo value to give the target quality parameter, comprises: determining segmentation data corresponding to the preset Qo value on the basis of the Qo value; and filtering the first parameters on the basis of the segmentation data to give the target quality parameter, such that the base filtering range of the target quality parameter matches the base filtering range of the preset Qo value. For example, according to the preset Qo value, the basecall error rate e corresponding to the preset Qo value can be acquired, and on the basis of the basecall error rate e, the first parameter satisfying the preset Qo value can be acquired. That is, the first parameter can also be processed according to this segmentation method to give the target quality parameter.

Research shows that the basecall error rate has a certain relationship with the quality parameter, and a high error rate may result from an excessively high or low quality parameter. Therefore, in order to give an accurate quality parameter for machine learning, the first parameter may be optimized to give a target quality parameter on the basis of the optimized first parameter. The examples of the present application further comprise:

> determining a basecall error rate corresponding to each of the first parameters;
> summarizing a first basecall error rate corresponding to first parameters greater than a preset first threshold on the basis of the basecall error rate corresponding to each of the first parameters, and summarizing a second basecall error rate corresponding to first parameters less than a preset second threshold;
> if the first basecall error rate and/or the second basecall error rate are/is greater than an error rate threshold, optimizing the first parameters on the basis of the basecall error rate corresponding to each of the first parameters to give a target first parameter;
> wherein, filtering the first parameters on the basis of the preset Qo value to give the target quality parameter, comprises:
> processing the target first parameter on the basis of the base filtering condition of the original quality parameter to give the target quality parameter.

Furthermore, determining the basecall error rate corresponding to each of the first parameters, comprises: calculating the basecall error rate via formula $Q = -10 \times \log_{10} e$, wherein Q is the calculated first parameter, and e is the basecall error rate.

Furthermore, optimizing the first parameters on the basis of the basecall error rate corresponding to each of the first parameters to give the target first parameter, comprises: updating the first parameter on the basis of the formula $Q_2 = -10 \times \log_{10}(2 \times P_{ID})$, wherein $Q_2$ denotes the target first parameter corresponding to the current first parameters, and $P_{ID}$ denotes the error rate corresponding to a set formed by the current first parameters. Therefore, the accuracy of the quality parameter corresponding to the first parameter can be fed back according to the error rate corresponding to the set formed by the current first parameters, and the current first parameters exceeding the preset error rate value are optimized, thereby improving the accuracy of the quality parameter and thus the accuracy of basecall.

Illustratively, the basecall was performed using a machine learning model on several sets of sequencing pictures (fov) from a group of experiments in which the complete standard human sample (HG001) human gene was completed using the latest fixed version of the sequencer and reagents, and the base prediction probabilities and parameters used for base prediction generated during this process were printed. The basecall result file was aligned with the human gene Hg19 reference and the correct bases corresponding to the predicted bases were acquired from the aligned sam file. Then, the machine learning basecall-printed intermediate parameters were assigned to the base corresponding to each probability and the bases on the reference genome, and the correctness or incorrectness of the probability was determined by whether the base types were consistent with the reference. For the correct bases, 1 was assigned to the corresponding maximum probability, and for the incorrect bases, 0 was assigned to the corresponding maximum probability. Two very important pieces of information were acquired by this point, the maximum probability of the bases and the correct and incorrect labels corresponding to the base probability. For example, value 1 was used to denote that the basecall result acquired on the basis of the basecall model is consistent with the true value of the label, or a value of 0 was used otherwise.

In the examples of the present application, to facilitate distinguishing between the original quality parameter and the target quality score, the original quality parameter may be characterized by the Qo value, and the target quality score may be characterized by the $Q_T$ value. Correspondingly, the algorithm for determining the original quality parameter is abbreviated as the Qo algorithm, and the algorithm for determining the target quality parameter is abbreviated as the $Q_T$ algorithm.

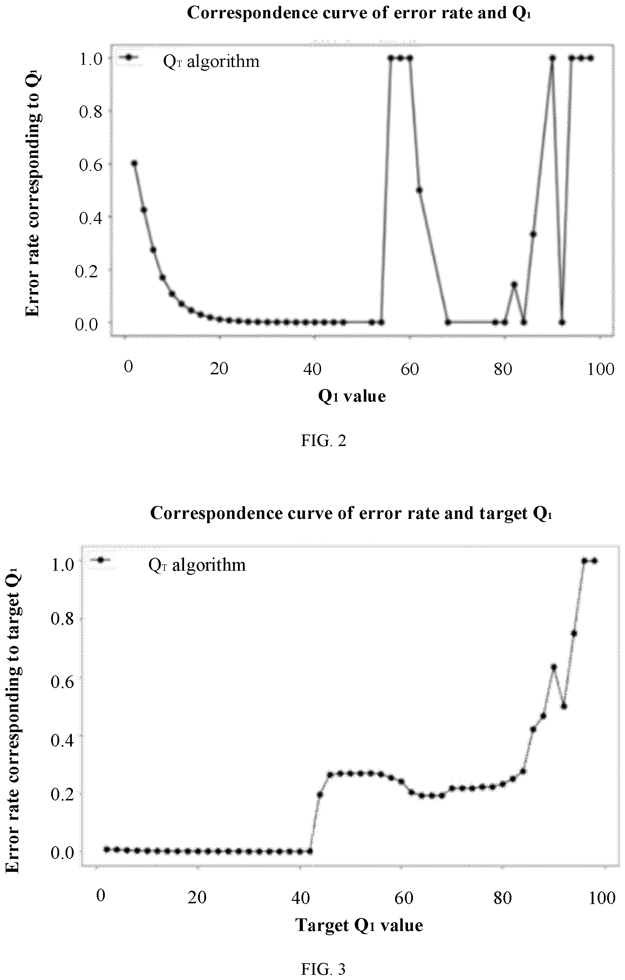

According to the acquired base probability distribution for a current base extension reaction in the nucleic acid sample of interest, numerical conversion is performed on the probability by using $Q_1 = -10 \times \log_{10}(1-P_{max})$ to convert the probability to a $Q_1$ value. The acquired $Q_1$ value is rounded to the nearest integer to give an integer $Q_1$ value. Then, error rate summarization is performed on all $Q_1$ values and error rate simulation calculation is performed on each $Q_1$ value, so as to give a correspondence schematic of the error rate and the $Q_1$ value as shown in FIG. 2. A curve closer to 0 denotes a lower error rate and thus a better classification. As can be seen from FIG. 2, the part with higher $Q_1$ values still exhibits great fluctuation and high error rates. Therefore, it is difficult to give an accurate quality score merely according to the $Q_1$ value. As such, the segmentation is necessary to separately calculate the Q value of the interval corresponding to each $Q_1$ value.

First, the $Q_1$ value data should be compared and summarized. Specifically, the error rates of the sets greater than a specific value (i.e., a preset first threshold) are summarized according to the calculation method of "the number of correct bases in the data greater than the preset $Q_1$/the total number of data greater than the preset $Q_1$". The curve given by the summarization result is shown in FIG. 3, where a curve closer to 0 in FIG. 3 denotes a lower error rate and thus a better filtering effect. In FIG. 3, the horizontal axis denotes the target first parameter, such as the target $Q_1$ value, and the vertical axis denotes the error rate of the bases greater than a calculated filtering threshold (i.e., greater than the preset first threshold), such as the error rate corresponding to the target $Q_1$ value.

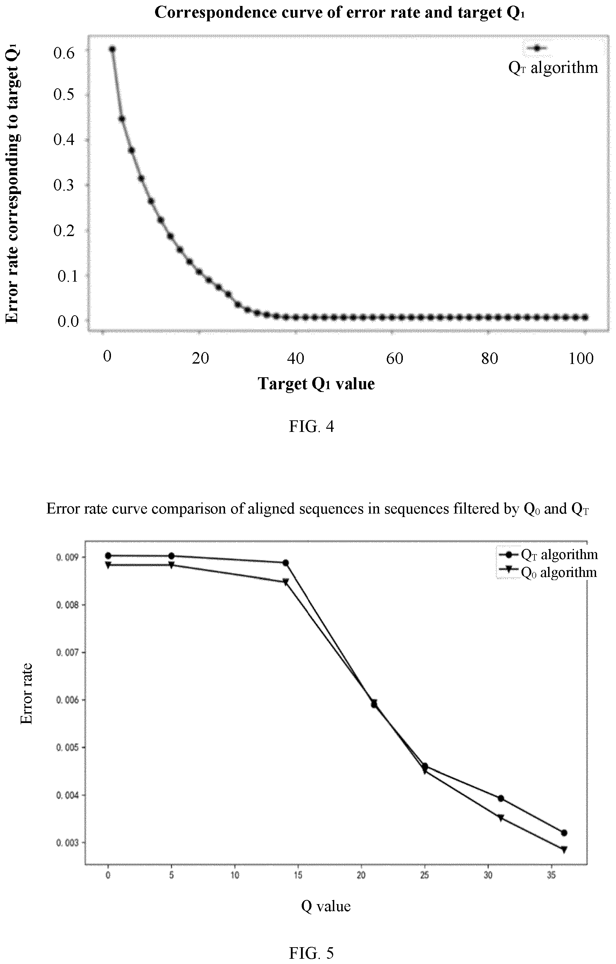

Specifically, the $Q_1$ values are summarized by summarizing the error rates of the sets less than a specific $Q_1$ value (e.g., a preset second threshold) according to the calculation method of "the number of correct bases in the data less than the preset $Q_1$/the total number of data less than the preset $Q_1$". The curve given by the summarization result is shown in FIG. 4, where a curve closer to 0 in FIG. 4 denotes a lower error rate and thus a better filtering effect. In FIG. 4, the horizontal axis denotes the target first parameter optimized by means of the preset second threshold, such as the target Q1 value, and the vertical axis denotes the error rate of the bases greater than a calculated filtering threshold (i.e., less than the preset second threshold), such as the error rate corresponding to the target Q1 value.

It can be seen that there is a certain relationship between the error rate and the magnitude of $Q_1$. A high error rate may result from an excessively high or low $Q_1$, and on this basis, a lower $Q_1$ value can be assigned to the $Q_1$ of such segment, whereas a higher $Q_T$ value can be assigned to a $Q_1$ interval with a low error rate. Furthermore, the error rate ($P_{ID}$) corresponding to the set formed by each $Q_1$ is subjected to Q value conversion and rounded to the nearest integer. The specific assignment method is as follows:

$$Q_2 = -10 \times \log_{10}(2 \times P_{ID})$$

wherein $Q_2$ denotes the target first parameter corresponding to the current first parameters, and $P_{ID}$ denotes the error rate corresponding to the set formed by the current first parameters.

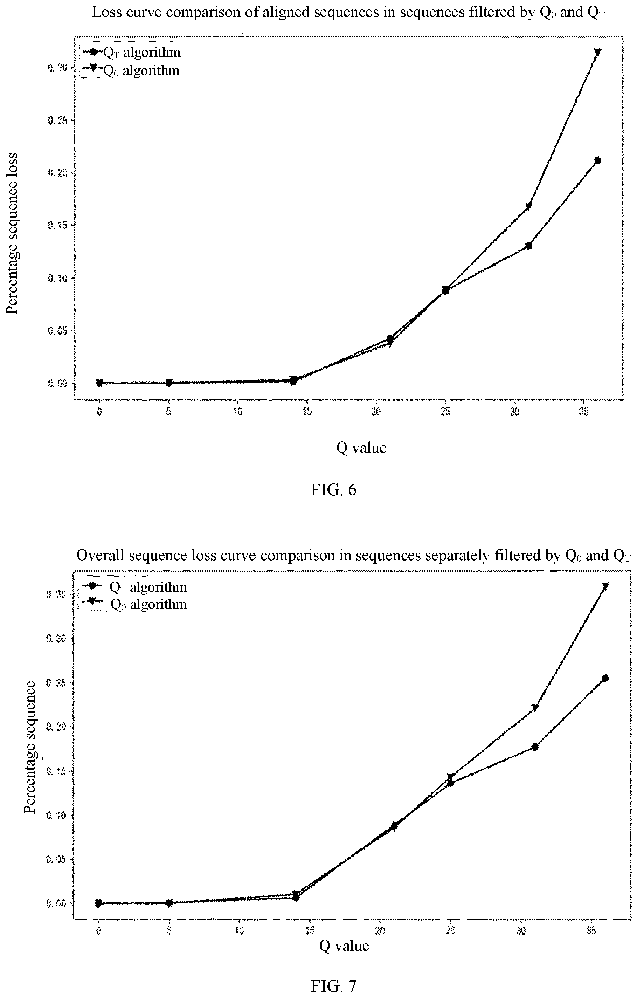

A more accurate $Q_T$ value is designed according to the classification error mode and the error rate reduction mode of $Q_0$, and the error rate and the filtered base loss are segmented and summarized. According to the error rate, the corresponding Q value of $Q_2$ and the conventional Qo can be acquired. In this way which part needs to be adjusted can be determined by comparing with Qo value. The best segment $Q_2$ can be found by searching. Herein, the comparison is performed by using Q_X_70-filtered data of the combination of Qz value with the machine-learning method or the combination of the preset Qo value with the conventional algorithm ("Q_X_70" filtering refers to that: when the proportion of bases having a Q value greater than or equal to an X value in the sequence is 70% or greater, the sequence is retained, and when the proportion of bases having a Q value greater than or equal to an X value in the sequence is less than 70%, the sequence is discarded; for example, for Q_25_70, when the proportion of bases having a Q value greater than or equal to 25 in the sequence is 70% or greater, the sequence is retained, and when the proportion of bases having a Q value greater than or equal to an 25 value in the sequence is less than 70%, the sequence is discarded). The related correspondence curves are shown in FIG. 5. A curve closer to 0 in FIG. 5 denotes a lower error rate and thus a better filtering effect. In FIG.5 the algorithm for determining the original quality parameter is abbreviated as the Qo algorithm, and the algorithm for determining the target quality parameter is abbreviated as the $Q_T$ algorithm. In FIG. 5, the abscissa denotes the Q value. The Q value in the curve corresponding to the Qo algorithm denotes the Qo value, and the Q value in the curve corresponding to the $Q_T$ algorithm denotes the $Q_T$ value. The ordinate in FIG. 5 denotes the error rate. Also referring to FIG. 6, a curve closer to 0 in FIG. 6 denotes fewer correct sequences being incorrectly discarded and thus a better filtering effect. In FIG. 6, the algorithm for determining the original quality parameter is abbreviated as the Qo algorithm, and the algorithm for determining the target quality parameter is abbreviated as the $Q_T$ algorithm. In FIG. 6, the abscissa denotes the Q value. The Q value in the curve corresponding to the Qo algorithm denotes the Qo value, and the Q value in the curve corresponding to the $Q_T$ algorithm denotes the $Q_T$ value. The ordinate in FIG. 6 denotes the percentage sequence loss. FIG. 7 illustrates the overall sequence loss curve comparison in sequences separately filtered by Qo value and $Q_T$ value. In FIG. 7, a curve closer to 0 denotes fewer discarded sequences, suggesting a lower sequence loss and thus a better filtering effect. As can be seen from FIG. 7, through segmentation, the sequence loss at the corresponding $Q_T$ value ensures that the error rate is superior or close to that of the conventional Qo value with acceptable throughput losses, demonstrating optimized results and a decreasing trend of the error rate.

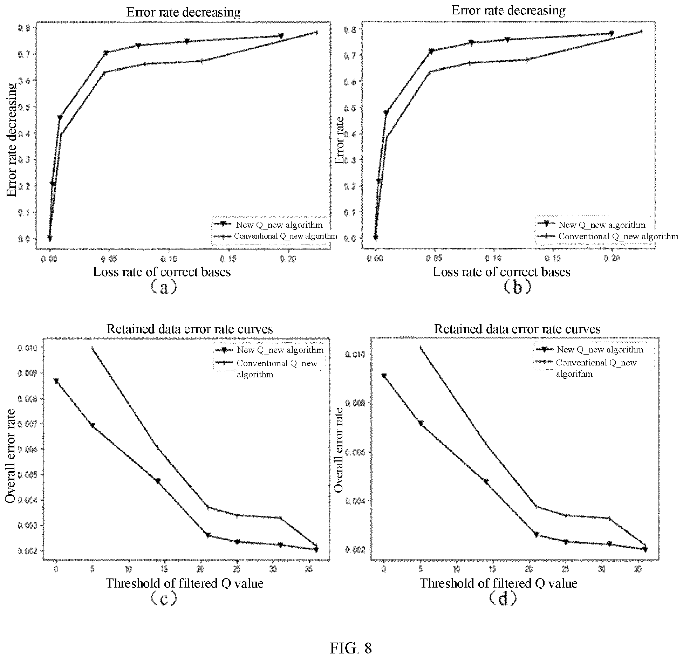

To ensure that the $Q_T$ values conform to the distribution of error rates of conventional Qo values, FIG. 8 is plotted using the same data according to the examples of the present application. As can be seen from panels (a), (b), (c), and (d) in FIG. 8, the error rate at the corresponding $Q_T$ values is consistent with the error rate at the conventional Qo values, but the $Q_T$ values have a lower base loss, which is compatible with the trend and results in the above drawings. As such, the $Q_T$ value segmentation can be determined as a real quality score segmentation and used as a target quality parameter of a machine learning model basecall algorithm. In FIG. 8, the new Q_new algorithm combination is the $Q_T$ algorithm, and the conventional Q_new algorithm is the Qo algorithm.

After the target quality parameter is acquired, the basecall result of the basecall model can be filtered on the basis of the target quality parameter to give a target basecall result.

The examples of the present application generate the target quality parameter on the basis of the calling result data of the basecall model, and apply the target quality parameter to the filtering of the basecall result of the corresponding basecall model. In one embodiment, the process of generating the basecall model comprises: acquiring training sample data comprising optical data of an original base channel; and taking the real basecall result corresponding to the training sample data as a training target, and training the training sample data to give a basecall model. The optical data may include data extracted directly from the fluorescent image acquired from the original base channel, such as fluorescence intensity values, or may include basecall results determined from the data acquired from the fluorescent image. The model structure of the basecall model may be a neural network model or a logistic regression model. For example, an isolation Forest model may be used. The real basecall results are labeled in the training sample to enable the prediction results to gradually approach the real basecall results in the training process until the corresponding threshold is met and the training ends.

In the examples of the present application, in addition to applying the maximum probability parameter in the current base extension reaction, the target quality parameter can also be determined by applying a qualified probability parameter. In one embodiment of the examples of the present application, calculating the first parameter on the basis of the base probability distribution, comprises:

acquiring test sample data of the basecall model and a predicted basecall result acquired by recognizing the test sample data on the basis of the basecall model, the test sample data comprising the real basecall result; determining a basecall probability screening condition on the basis of a correspondence relationship between the predicted basecall result and the real basecall result; and performing probability parameter screening in the probability distribution on the basis of the basecall probability screening condition to give the first parameter.

Furthermore, determining the basecall probability screening condition on the basis of a correspondence relationship between the predicted basecall result and the real basecall result, comprises:

acquiring data in which the predicted basecall result of the current base extension reaction is consistent with the real basecall result in each test sample data, and summarizing a base prediction probability of the corresponding current base reaction in the data; and determining the screening range of the basecall probability on the basis of the prediction probability.

For example, when the base probability distribution of a site corresponding to the current base extension reaction is [0.1,0.2,0.4,0.3], the maximum probability of the site is 0.4, and the base type corresponding to the maximum probability is base C. The real basecall result of this site is also base C. When the statistical prediction result is consistent with the real base result, the range of the probability range is usually concentrated on, for example, 0.4 to 0.6. A higher or lower range may indicate an issue with the sample prediction. This screening range is used as the probability parameter of the subsequent applications to calculate the target quality parameter. Specifically, this embodiment differs from the above examples in the probability parameter only, with the subsequent processing procedures similar to those applying the maximum probability parameter, which are not recited in detail.

The following describes the method for determining a quality parameter in basecall according to the examples of the present application in a specific application scenario and the effect of filtering the basecall results.

The data employed in this application scenario come from the following procedures:

The basecall was performed using a machine learning model basecall on several sets of sequencing pictures (fov) from a group of experiments in which the complete standard human sample (HG001) human gene was completed using the latest fixed version of the sequencer and reagents, and the base prediction probabilities and parameters used for base prediction generated during this process were printed. The basecall result file was aligned with the human gene Hg19 reference and the correct bases corresponding to the predicted bases were acquired from the aligned sam file. Then, the machine learning basecall-printed intermediate parameters were assigned to the base corresponding to each probability and the bases on the reference genome, and the correctness or incorrectness of the probability was determined by whether the base types were consistent with the reference. For the correct bases, 1 was assigned to the corresponding maximum probability, and for the incorrect bases, 0 was assigned to the corresponding maximum probability. Two very important pieces of information were acquired by this point, the maximum probability of the bases and the correct and incorrect labels corresponding to the base probability (values 1 and 0).

According to the acquired data, numerical conversion is performed on the probability to give a second parameter $Q_1 = -10 \times \log_{10}(1-P_{max})$. The second parameter is compared with a preset value. If the second parameter is greater than the preset value, the second parameter is rounded to the nearest integer toward zero to give the first parameter; if the second parameter is not greater than the preset value, the second parameter is rounded to the nearest integer to give the first parameter, wherein the range of the first parameter is positive integers from 0 to the preset value. Specifically, $Q_1$ is

rounded to give an integer value and the maximum of the value is limited to 100 (the value is assigned 100 if it exceeds 100). Then, the probability and the first parameter are taken as data feature values (that is, the data are input data of an input model). Probability values using 0 and 1 to denote incorrectness and correctness are put into an isolated Forest model for fitting, and therefore the correct and incorrect sets are distinguished to give the base probability.

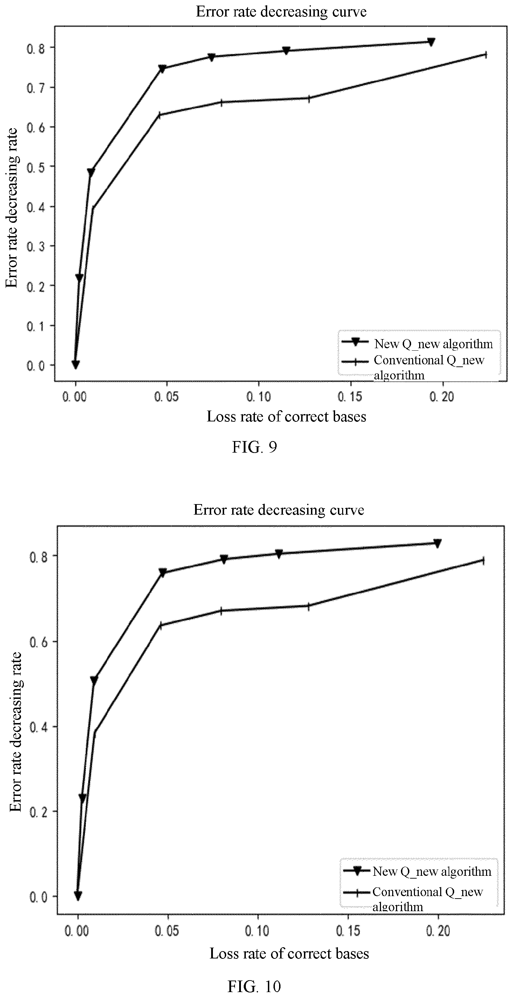

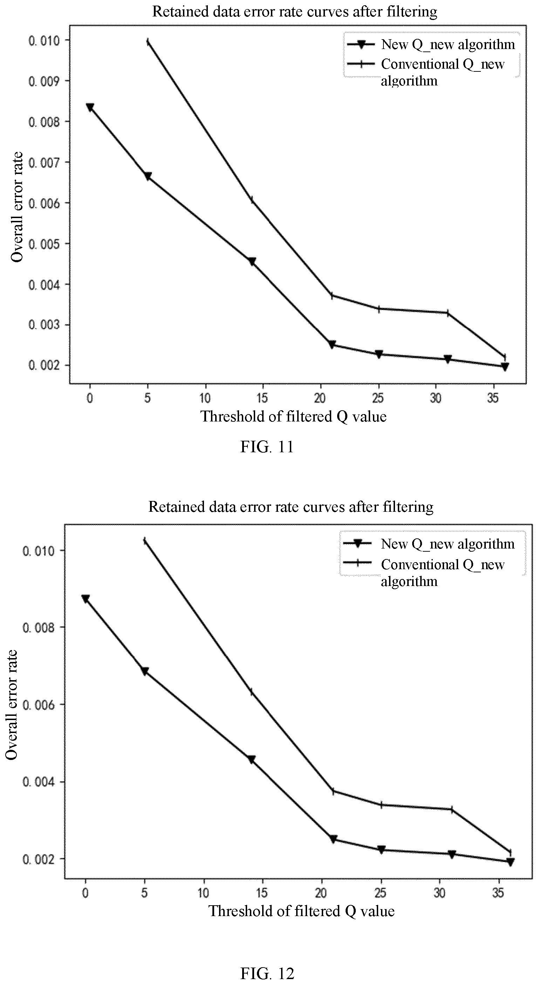

The division by probability is mainly intended to segment the region where the bases are positioned so as to better form a correspondence relationship with the conventional Qo value (i.e., the original quality parameter in the examples of the present application), predict and summarize the division condition using the homologous data completely irrelevant to the above training data, and divide the probability value into intervals, thus giving FIGs. 9 to 12. As can be seen from FIGs. 9 to 12, compared with the conventional Qo value, the segmentation using the target quality parameter is superior, with a lower error rate. This is a preferred option in practical application. It should be noted that in FIGs. 9 to 12, the new Q_new algorithm is the $Q_T$ algorithm, and the conventional Q_new algorithm is the Qo algorithm. Correspondingly, the threshold for filtered Q value refers to the target first parameter acquired by optimizing the first parameters with the data acquired by filtering the error rates with the preset first threshold or the preset second threshold. The specific implementation details are described in the foregoing examples.

The examples of the present application further provide an apparatus for determining a quality parameter in basecall, which can be used in the method for determining a quality parameter in basecall.

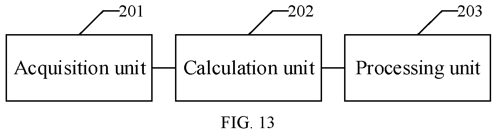

Referring to FIG. 13, the apparatus for determining the quality parameter in basecall is further provided comprises:

an acquisition unit 201, configured for acquiring calling result data of a basecall model for a nucleic acid sample of interest, the calling result data comprising base probability distribution for a current base extension reaction in the nucleic acid sample of interest;
a calculation unit 202, configured for calculating a first parameter on the basis of the base probability distribution; and
a processing unit 203, configured for filtering the first parameters on the basis of a preset Qo value to give a target quality parameter, wherein Qo = -10 $\times$ $\log_{10}$e, and e is a basecall error rate.

Optionally, the calculation unit comprises:

a first acquisition subunit, configured for acquiring a maximum probability parameter in the current base extension reaction on the basis of the base probability distribution; and

a first calculation subunit, configured for calculating the first parameter on the basis of the maximum probability parameter.

Optionally, the first calculation subunit is specifically configured for:

determining a second parameter $Q_1$ on the basis of formula $Q_1 = -10\times\log_{10} (1-P_{max})$, wherein $P_{max}$ denotes the maximum probability parameter;
comparing the second parameter with a preset value, and if the second parameter is greater than the preset value, rounding the second parameter to the nearest integer toward zero to give the first parameter; and if the second parameter is not greater than the preset value, rounding the second parameter to the nearest integer to give the first parameter, wherein the range of the first parameter is positive integers from 0 to the preset value.

Optionally, the apparatus further comprises:

a first determination unit, configured for determining a basecall error rate corresponding to each of the first parameters;
a summarizing unit, configured for summarizing a first basecall error rate corresponding to first parameters greater than a preset first threshold on the basis of the basecall error rate corresponding to each of the first parameters, and summarizing a second basecall error rate corresponding to first parameters less than a preset second threshold;
an optimization unit, configured for, if the first basecall error rate and/or the second basecall error rate are/is greater than an error rate threshold, optimizing the first parameters on the basis of the basecall error rate corresponding to each of the first parameters to give a target first parameter;
wherein the processing unit is specifically configured for:
filtering the target first parameters on the basis of the preset Qo value to give the target quality parameter.

Optionally, the optimization unit is specifically configured for:
updating the first parameter on the basis of the formula $Q_2 = -10 \times \log_{10}(2\times P_{ID})$, wherein $Q_2$ denotes the target first parameter corresponding to the current first parameters, and $P_{ID}$ denotes the error rate corresponding to a set formed by the current first parameters.

Optionally, the processing unit comprises:

a determination subunit, configured for determining segmentation data corresponding to the preset Qo value on the basis of the preset Qo value; and
a processing subunit, configured for filtering the first parameters on the basis of the segmentation data to give the target quality parameter, such that the base filtering range of the target quality parameter matches the base filtering range of the Qo value.

Optionally, the apparatus further comprises:
a filtering unit, configured for filtering the basecall results of the basecall model on the basis of the target quality parameter to give a target basecall result.

Optionally, the apparatus further comprises a model training unit configured for:

acquiring training sample data comprising optical data of an original base channel; and
taking the real basecall result corresponding to the training sample data as a training target, and training the training sample data to give a basecall model.

Optionally, the calculation unit comprises:

a calling subunit, configured for acquiring test sample data of the basecall model and a predicted basecall result acquired by recognizing the test sample data on the basis of the basecall model, the test sample data comprising the real basecall result;
a second determination subunit, configured for determining a basecall probability screening condition on the basis of a correspondence relationship between the predicted basecall result and the real basecall result; and
a screening subunit, configured for performing probability parameter screening in the probability distribution on the basis of the basecall probability screening condition to give the first parameter.

Optionally, the second determination subunit is specifically configured for:

acquiring data in which the predicted basecall result of the current base extension reaction is consistent with the real basecall result in each test sample data, and summarizing a base prediction probability of the corresponding current base reaction in the data; and
determining the screening range of the basecall probability on the basis of the prediction probability.

It should be noted that, in this example, reference may be made to the corresponding disclosure in the foregoing for the specific implementations of each unit and sub-unit, which will not be recited here.

In another example of the present application, further provided is a readable storage medium having a computer program stored thereon, the computer program, when executed by a processor, implementing the method for determining a quality parameter in basecall as described in any one of those above.

In another example of the present application, further provided is an electronic device, comprising:

a memory, configured for storing an application and data generated by the operation of the application; and
a processor, configured for executing the application to implement the method for determining a quality parameter in basecall as described in any one of those above.

It should be noted that, in this example, reference may be made to the corresponding disclosure in the foregoing for the specific implementations of the processor, which will not be recited here.

In the specification, the examples are described in a progressive manner. The examples focus on their differences from other examples, and reference can be made among other examples for the same and similar parts. Since the apparatus disclosed in the examples corresponds to the method disclosed in the examples, the description of the apparatus is simplified, and reference can be made to the description of the method for the relevant part.

Those skilled in the art would further appreciate that the various exemplary units and algorithm procedures described in the examples of the present application may be implemented as electronic hardware, computer software, or a combination of the two, and that the components and procedures of the various examples have been described above generally in terms of their functionality in order to clearly illustrate this interchangeability of hardware and software. Whether such functionality is implemented as hardware or software depends upon the particular application and design constraints imposed on the technical solution. Those skilled in the art may implement the described functionality in varying ways for each particular application, but such implementation decisions should not be construed as causing a departure from the scope of the present application.

The procedures of a method or algorithm described in the examples of the present application may be implemented directly in hardware, in a software module executed by a processor, or in a combination of the two. The software module may reside in a random access memory (RAM), memory, read-only memory (ROM), electrically programmable ROM, electrically erasable programmable ROM, register, hard disk, removable disk, CD-ROM, or any other form of storage medium known in the art.

The above description of the disclosed examples is provided to enable those skilled in the art to implement or use the present application. Various modifications to these examples will be readily apparent to those skilled in the art, and the generic principles defined herein may be applied to other examples without departing from the spirit or scope of the present application. Thus, the present application is limited to the examples illustrated herein but is in accordance with the broadest scope consistent with the principles and novel features disclosed herein.

The present invention may be characterized by the following items.

1. A method for determining a quality parameter in basecall, comprising:

acquiring calling result data of a basecall model for a nucleic acid sample of interest, the calling result data comprising base probability distribution for a current base extension reaction in the nucleic acid sample of interest;
calculating a first parameter on the basis of the base probability distribution; and
filtering the first parameters on the basis of a preset Qo value to give a target quality parameter, wherein Qo = -10 × $\log_{10}$e, and e is a basecall error rate.

2. The method according to item 1, wherein, calculating the first parameter on the basis of the base probability distribution, comprises:

acquiring a maximum probability parameter in the current base extension reaction on the basis of the base probability distribution; and
calculating the first parameter on the basis of the maximum probability parameter.

3. The method according to item 2, wherein, calculating the first parameter on the basis of the maximum probability parameter, comprises:

determining a second parameter $Q_1$ on the basis of formula $Q_1 = -10 \times \log_{10} (1-P_{max})$, wherein $P_{max}$ denotes the maximum probability parameter;
comparing the second parameter with a preset value, and if the second parameter is greater than the preset value, rounding the second parameter to the nearest integer toward zero to give the first parameter; and
if the second parameter is not greater than the preset value, rounding the second parameter to the nearest integer to give the first parameter, wherein the range of the first parameter is positive integers from 0 to the preset value.

4. The method according to any one of items 1-3, further comprising:

determining a basecall error rate corresponding to each of the first parameters;
summarizing a first basecall error rate corresponding to first parameters greater than a preset first threshold on the basis of the basecall error rate corresponding to each of the first parameters, and summarizing a second basecall error rate corresponding to first parameters less than a preset second threshold;

if the first basecall error rate and/or the second basecall error rate are/is greater than an error rate threshold, optimizing the first parameters on the basis of the basecall error rate corresponding to each of the first parameters to give a target first parameter;
wherein, filtering the first parameters on the basis of the preset Qo value to give the target quality parameter, comprises:
filtering the target first parameters on the basis of the preset Qo value to give the target quality parameter.

5. The method according to item 4, wherein, optimizing the first parameters on the basis of the basecall error rate corresponding to each of the first parameters to give the target first parameter, comprises: updating the first parameter on the basis of the formula $Q_2 = -10 \times \log_{10}(2 \times P_{ID})$, wherein $Q_2$ denotes the target first parameter corresponding to the current first parameters, and $P_{ID}$ denotes the error rate corresponding to a set formed by the current first parameters.

6. The method according to any one of items 1-5, wherein, filtering the first parameters on the basis of the preset $Q_0$ value to give the target quality parameter, comprises:

determining segmentation data corresponding to the preset Qo value on the basis of the preset Qo value; and
filtering the first parameters on the basis of the segmentation data to give the target quality parameter, such that the base filtering range of the target quality parameter matches the base filtering range of the Qo value.

7. The method according to any one of items 1-6, further comprising:
filtering basecall results of the basecall model on the basis of the target quality parameter to give a target basecall result.

8. The method according to any one of items 1-7, further comprising:

acquiring training sample data comprising optical data of an original base channel; and
taking the real basecall result corresponding to the training sample data as a training target, and training the training sample data to give a basecall model.

9. The method according to any one of items 1-8, wherein, calculating the first parameter on the basis of the base probability distribution, comprises:

acquiring test sample data of the basecall model and a predicted basecall result acquired by recognizing the test sample data on the basis of the basecall model, the test sample data comprising the real basecall result; determining a basecall probability screening condition on the basis of a correspondence relationship between the predicted basecall result and the real basecall result; and

performing probability parameter screening in the probability distribution on the basis of the basecall probability screening condition to give the first parameter.

10. The method according to item 9, wherein, determining the basecall probability screening condition on the basis of the correspondence relationship between the predicted basecall result and the real basecall result, comprises:

acquiring data in which the predicted basecall result of the current base extension reaction is consistent with the real basecall result in each test sample data, and summarizing a base prediction probability of the corresponding current base reaction in the data; and

determining the screening range of the basecall probability on the basis of the prediction probability.

11. An apparatus for determining a quality parameter in basecall, comprising:

an acquisition unit, configured for acquiring calling result data of a basecall model for a nucleic acid sample of interest, the calling result data comprising base probability distribution for a current base extension reaction in the nucleic acid sample of interest;

a calculation unit, configured for calculating a first parameter on the basis of the base probability distribution; and

a processing unit, configured for filtering the first parameters on the basis of a preset Qo value to give a target quality parameter, wherein $Qo = -10 \times \log_{10} e$, and e is a basecall error rate.

12. The apparatus according to item 11, wherein the calculation unit comprises:

a first acquisition subunit, configured for acquiring a maximum probability parameter in the current base extension reaction on the basis of the base probability distribution; and

a first calculation subunit, configured for calculating the first parameter on the basis of the maximum probability parameter.

13. The apparatus according to item 12, wherein the first calculation subunit is specifically configured for:

determining a second parameter $Q_1$ on the basis of formula $Q_1 = -10 \times \log_{10} (1-P_{max})$, wherein $P_{max}$ denotes the maximum probability parameter;

comparing the second parameter with a preset value, and if the second parameter is greater than the preset value, rounding the second parameter to the nearest integer toward zero to give the first parameter; and

if the second parameter is not greater than the preset value, rounding the second parameter to the nearest integer to give the first parameter, wherein the range of the first parameter is positive integers from 0 to the preset value.

14. The apparatus according to any one of items 11-13, further comprising:

a first determination unit, configured for determining a basecall error rate corresponding to each of the first parameters;

a summarizing unit, configured for summarizing a first basecall error rate corresponding to first parameters greater than a preset first threshold on the basis of the basecall error rate corresponding to each of the first parameters, and summarizing a second basecall error rate corresponding to first parameters less than a preset second threshold;

an optimization unit, configured for, if the first basecall error rate and/or the second basecall error rate are/is greater than an error rate threshold, optimizing the first parameters on the basis of the basecall error rate corresponding to each of the first parameters to give a target first parameter;

wherein the processing unit is specifically configured for:

filtering the target first parameters on the basis of the preset Qo value to give the target quality parameter.

15. The apparatus according to item 14, wherein the optimization unit is specifically configured for:

updating the first parameter on the basis of the formula $Q_2 = -10 \times \log_{10}(2 \times P_{ID})$, wherein $Q_2$ denotes the target first parameter corresponding to the current first parameters, and $P_{ID}$ denotes the error rate corresponding to a set formed by the current first parameters.

16. The apparatus according to any one of items 11-15, wherein the processing unit comprises:

a determination subunit, configured for determining segmentation data corresponding to the preset Qo value on the basis of the preset Qo value; and

a processing subunit, configured for filtering the first parameters on the basis of the segmentation data to give the target quality parameter, such that the base filtering range of the target quality parameter matches the base filtering range of the Qo value.

17. The apparatus according to any one of items 11-16, further comprising
a filtering unit, configured for filtering the basecall results of the basecall model on the basis of the target quality parameter to give a target basecall result.

18. The apparatus according to any one of items 11-17, further comprising a model training unit configured for:

acquiring training sample data comprising optical data of an original base channel; and
taking the real basecall result corresponding to the training sample data as a training target, and training the training sample data to give a basecall model.

19. The apparatus according to any one of items 11-18, wherein the calculation unit comprises:

a calling subunit, configured for acquiring test sample data of the basecall model and a predicted basecall result acquired by recognizing the test sample data on the basis of the basecall model, the test sample data comprising the real basecall result;
a second determination subunit, configured for determining a basecall probability screening condition on the basis of a correspondence relationship between the predicted basecall result and the real basecall result; and
a screening subunit, configured for performing probability parameter screening in the probability distribution on the basis of the basecall probability screening condition to give the first parameter.

20. The apparatus according to item 19, wherein the second determination subunit is specifically configured for:

acquiring data in which the predicted basecall result of the current base extension reaction is consistent with the real basecall result in each test sample data, and summarizing a base prediction probability of the corresponding current base reaction in the data; and
determining the screening range of the basecall probability on the basis of the prediction probability.

**Claims**

1. A method for determining a quality parameter in basecall, comprising:

acquiring calling result data of a basecall model for a nucleic acid sample of interest, the calling result data comprising base probability distribution for a current base extension reaction in the nucleic acid sample of interest;
calculating a first parameter on the basis of the base probability distribution; and
filtering the first parameters on the basis of a preset Qo value to give a target quality parameter, wherein $Qo = -10 \times \log_{10} e$, and e is a basecall error rate.

2. The method according to claim 1, wherein, calculating the first parameter on the basis of the base probability distribution, comprises:

acquiring a maximum probability parameter in the current base extension reaction on the basis of the base probability distribution; and
calculating the first parameter on the basis of the maximum probability parameter;
optionally, wherein, calculating the first parameter on the basis of the maximum probability parameter, comprises:

determining a second parameter $Q_1$ on the basis of formula $Q_1 = -10 \times \log_{10} (1-P_{max})$, wherein $P_{max}$ denotes the maximum probability parameter;
comparing the second parameter with a preset value, and if the second parameter is greater than the preset value, rounding the second parameter to the nearest integer toward zero to give the first parameter; and if the second parameter is not greater than the preset value, rounding the second parameter to the nearest integer to give the first parameter, wherein the range of the first parameter is positive integers from 0 to the preset value.

3. The method according to claim 1 or 2, further comprising:

determining a basecall error rate corresponding to each of the first parameters;
summarizing a first basecall error rate corresponding to first parameters greater than a preset first threshold on the basis of the basecall error rate corresponding to each of the first parameters, and summarizing a second basecall error rate corresponding to first parameters less than a preset second threshold;

if the first basecall error rate and/or the second basecall error rate are/is greater than an error rate threshold, optimizing the first parameters on the basis of the basecall error rate corresponding to each of the first parameters to give a target first parameter;

wherein, filtering the first parameters on the basis of the preset Qo value to give the target quality parameter, comprises:

filtering the target first parameters on the basis of the preset Qo value to give the target quality parameter; optionally, wherein, optimizing the first parameters on the basis of the basecall error rate corresponding to each of the first parameters to give the target first parameter, comprises:

updating the first parameter on the basis of the formula $Q_2 = -10 \times \log_{10}(2 \times P_{ID})$, wherein $Q_2$ denotes the target first parameter corresponding to the current first parameters, and $P_{ID}$ denotes the error rate corresponding to a set formed by the current first parameters.

4. The method according to any one of claims 1-3, wherein, filtering the first parameters on the basis of the preset $Q_0$ value to give the target quality parameter, comprises:
determining segmentation data corresponding to the preset Qo value on the basis of the preset Qo value; and filtering the first parameters on the basis of the segmentation data to give the target quality parameter, such that the base filtering range of the target quality parameter matches the base filtering range of the Qo value.

5. The method according to any one of claims 1-4, further comprising:
filtering basecall results of the basecall model on the basis of the target quality parameter to give a target basecall result.

6. The method according to any one of claims 1-5, further comprising:

acquiring training sample data comprising optical data of an original base channel; and taking the real basecall result corresponding to the training sample data as a training target, and training the training sample data to give a basecall model.

7. The method according to any one of claims 1-6, wherein, calculating the first parameter on the basis of the base probability distribution, comprises:

acquiring test sample data of the basecall model and a predicted basecall result acquired by recognizing the test sample data on the basis of the basecall model, the test sample data comprising the real basecall result;
determining a basecall probability screening condition on the basis of a correspondence relationship between the predicted basecall result and the real basecall result; and
performing probability parameter screening in the probability distribution on the basis of the basecall probability screening condition to give the first parameter;
optionally, wherein, determining the basecall probability screening condition on the basis of the correspondence relationship between the predicted basecall result and the real basecall result, comprises:

acquiring data in which the predicted basecall result of the current base extension reaction is consistent with the real basecall result in each test sample data, and summarizing a base prediction probability of the corresponding current base reaction in the data; and
determining the screening range of the basecall probability on the basis of the prediction probability.

8. An apparatus for determining a quality parameter in basecall, comprising:

an acquisition unit, configured for acquiring calling result data of a basecall model for a nucleic acid sample of interest, the calling result data comprising base probability distribution for a current base extension reaction in the nucleic acid sample of interest;
a calculation unit, configured for calculating a first parameter on the basis of the base probability distribution; and
a processing unit, configured for filtering the first parameters on the basis of a preset Qo value to give a target quality parameter, wherein Qo = -10 $\times \log_{10}e$, and e is a basecall error rate.

9. The apparatus according to claim 8, wherein the calculation unit comprises:

a first acquisition subunit, configured for acquiring a maximum probability parameter in the current base extension reaction on the basis of the base probability distribution; and
a first calculation subunit, configured for calculating the first parameter on the basis of the maximum probability parameter;
optionally, wherein the first calculation subunit is specifically configured for:

determining a second parameter $Q_1$ on the

basis of formula $Q_1 = -10 \times \log_{10}(1-P_{max})$, wherein $P_{max}$ denotes the maximum probability parameter;

comparing the second parameter with a preset value, and if the second parameter is greater than the preset value, rounding the second parameter to the nearest integer toward zero to give the first parameter; and if the second parameter is not greater than the preset value, rounding the second parameter to the nearest integer to give the first parameter, wherein the range of the first parameter is positive integers from 0 to the preset value.

10. The apparatus according to claim 8 or 9, further comprising:

a first determination unit, configured for determining a basecall error rate corresponding to each of the first parameters;
a summarizing unit, configured for summarizing a first basecall error rate corresponding to first parameters greater than a preset first threshold on the basis of the basecall error rate corresponding to each of the first parameters, and summarizing a second basecall error rate corresponding to first parameters less than a preset second threshold;
an optimization unit, configured for, if the first basecall error rate and/or the second basecall error rate are/is greater than an error rate threshold, optimizing the first parameters on the basis of the basecall error rate corresponding to each of the first parameters to give a target first parameter;
wherein the processing unit is specifically configured for:
filtering the target first parameters on the basis of the preset Qo value to give the target quality parameter; optionally, wherein the optimization unit is specifically configured for:
updating the first parameter on the basis of the formula $Q_2 = -10 \times \log_{10}(2 \times P_{ID})$, wherein $Q_2$ denotes the target first parameter corresponding to the current first parameters, and $P_{ID}$ denotes the error rate corresponding to a set formed by the current first parameters.

11. The apparatus according to any one of claims 8-10, wherein the processing unit comprises:

a determination subunit, configured for determining segmentation data corresponding to the preset Qo value on the basis of the preset Qo value; and
a processing subunit, configured for filtering the first parameters on the basis of the segmentation data to give the target quality parameter, such that the base filtering range of the target quality parameter matches the base filtering range of the Qo value.

12. The apparatus according to any one of claims 8-11, further comprising
a filtering unit, configured for filtering the basecall results of the basecall model on the basis of the target quality parameter to give a target basecall result.

13. The apparatus according to any one of claims 8-12, further comprising a model training unit configured for:

acquiring training sample data comprising optical data of an original base channel; and
taking the real basecall result corresponding to the training sample data as a training target, and training the training sample data to give a basecall model.

14. The apparatus according to any one of claims 8-13, wherein the calculation unit comprises:

a calling subunit, configured for acquiring test sample data of the basecall model and a predicted basecall result acquired by recognizing the test sample data on the basis of the basecall model, the test sample data comprising the real basecall result;
a second determination subunit, configured for determining a basecall probability screening condition on the basis of a correspondence relationship between the predicted basecall result and the real basecall result; and
a screening subunit, configured for performing probability parameter screening in the probability distribution on the basis of the basecall probability screening condition to give the first parameter;
optionally, wherein the second determination subunit is specifically configured for:

acquiring data in which the predicted basecall result of the current base extension reaction is consistent with the real basecall result in each test sample data, and summarizing a base prediction probability of the corresponding current base reaction in the data; and
determining the screening range of the basecall probability on the basis of the prediction probability.

Acquire calling result data of a basecall model for a sample of interest — S101

Calculate a first parameter on the basis of the base probability distribution — S102

Process the first parameter on the basis of the base filtering condition of the original quality parameter to give the target quality parameter — S103

FIG. 1

## Correspondence curve of error rate and $Q_1$

FIG. 2

## Correspondence curve of error rate and target $Q_1$

FIG. 3

## Correspondence curve of error rate and target $Q_1$

FIG. 4

Error rate curve comparison of aligned sequences in sequences filtered by $Q_0$ and $Q_T$

FIG. 5

Loss curve comparison of aligned sequences in sequences filtered by $Q_0$ and $Q_T$

FIG. 6

Overall sequence loss curve comparison in sequences separately filtered by $Q_0$ and $Q_T$

FIG. 7

FIG. 8

EP 4 576 102 A1

Error rate decreasing curve

FIG. 9

Error rate decreasing curve

FIG. 10

21

Retained data error rate curves after filtering

FIG. 11

Retained data error rate curves after filtering

FIG. 12

201
202
203

| Acquisition unit | Calculation unit | Processing unit |

FIG. 13

Europäisches
Patentamt

European
Patent Office

Office européen
des brevets

# EUROPEAN SEARCH REPORT

**Application Number**

EP 24 22 2357

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/044229 A1 (ILLUMINA INC [US]; ILLUMINA SOFTWARE INC [US]) 23 March 2023 (2023-03-23) * abstract * * paragraph [0021] - paragraph [0104] * * paragraph [0120] - paragraph [0159] * * claims 1-5,17-20 * * figures 1,2 * ----- | 1-14 | INV. G16B40/20 G16B30/00 |

TECHNICAL FIELDS
SEARCHED        (IPC)

G16B

The present search report has been drawn up for all claims

| Place of search | Date of completion of the search | Examiner |
|---|---|---|
| Munich | 18 May 2025 | Hilbig, Matthias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 24 22 2357

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2025

| Patent document cited in search report | Publication date | Patent family member(s) | | Publication date |
|---|---|---|---|---|
| WO 2023044229 A1 | 23-03-2023 | CN | 117561573 A | 13-02-2024 |
| | | EP | 4402682 A1 | 24-07-2024 |
| | | JP | 2024535663 A | 02-10-2024 |
| | | US | 2023093253 A1 | 23-03-2023 |
| | | WO | 2023044229 A1 | 23-03-2023 |

EPO FORM P0459

For more details about this annex : see Official Journal of the European Patent Office, No. 12/82